# EUROPÄISCHE PATENTANMELDUNG

(11) **EP 1 267 297 A2**
(43) Veröffentlichungstag der Anmeldung: **18.12.2002**
(21) Anmeldenummer: 02012117.4
(22) Anmeldetag: 31.05.2002
(51) Int. Cl.: G06F 19/00

(54) **Verfahren zur Steuerung und Überwachung des Prozessablaufs einer zu erbringenden telemedizinischen Gesundheitsdienstleistung**

(30) Priorität: 13.06.2001 DE 10128522
(71) Anmelder: SIEMENS AKTIENGESELLSCHAFT, 80333 München (DE)
(72) Erfinder: Christ, Tilo, 91058 Erlangen (DE); Prihoda, Heinz, 90562 Heroldsberg (DE); Schmidt, Volker, Dr., 91054 Erlangen (DE); Schneider, Siegfried, Dr., 91056 Erlangen (DE); Schüll, Hans-Dieter, 91085 Weisendorf (DE); Striebel, Werner, 90592 Schwarzenbruck (DE); Zahlmann, Gudrun, Dr., 92318 Neumarkt (DE)

(57) **Zusammenfassung**

Die Erfindung betrifft ein Verfahren zur Steuerung und Überwachung des Prozessablaufs einer zu erbringenden telemedizinischen Gesundheitsdienstleistung durch eine Datenverarbeitungseinrichtung (6). Basierend auf wenigstens einem Kriterium erfolgt eine automatisierte Beauftragung einer zur Erbringung einer bestimmten Teilleistung der Gesundheitsdienstleistung zur Verfügung stehenden Dienstleistungseinrichtung (1 bis 4) zur Erbringung der bestimmten Teilleistung durch die zentrale Datenverarbeitungseinrichtung (6). Die zentrale Datenverarbeitungseinrichtung (6) überwacht anschließend, ob die jeweilige Teilleistung entsprechend einer zeitlichen Vorgabe rechtzeitig von der beauftragten Dienstleistungseinrichtung (1 bis 4) erbracht und an die zentrale Datenverarbeitungseinrichtung (6) übermittelt wird, und/oder ob die erbrachte jeweilige Teilleistung einer qualitativen Vorgabe genügt.

## Beschreibung

Im Zuge der Behandlung und medizinischen Betreuung von Patienten gewinnen telemedizinische Gesundheitsdienstleistungen zunehmend an Bedeutung. Unter telemedizinischen Gesundheitsdienstleistungen werden dabei jegliche Arten von Telemedizin verstanden, beispielsweise die Befundung von medizinischen Datensätzen, welche an eine Dienstleistungseinrichtung für telemedizinische Gesundheitsdienstleistungen über ein Kommunikationsnetz übersandt wurden, oder die Online-Befundung von Körperfunktionen unter Zuhilfenahme von technischen Mittel zur Fernübertragung von Daten. Telemedizinische Gesundheitsdienstleistungen werden also vorzugsweise komplett von der Auftragsvergabe, über die Ausführung bis zur Lieferung des Ergebnisses unter Zuhilfenahme von Mittel zur Fernübertragung von Daten abgewickelt.

Eine Gesundheitsdienstleistung, welche in der Regel mehrere Teilleistungen beinhaltet, beispielsweise die Aufnahme von Bilddaten und die Auswertung von Bilddaten, wird zumeist nicht von einer einzigen Dienstleistungseinrichtung, worunter beispielsweise niedergelassene Ärzte, also Hausärzte und Fachärzte, Kliniken sowie spezielle Bildaufnahme- oder Befundungszentren zu verstehen sind, bearbeitet. Vielmehr erfolgt die Erbringung einer telemedizinischen Gesundheitsdienstleistung zumeist durch mehrere derartige Dienstleistungseinrichtungen, welche eine Gesundheitsdienstleistungskette bilden. Aufgrund der Verteilung von Teilleistungen der im Rahmen einer telemedizinischen Gesundheitsdienstleistung zu erbringenden Gesamtleistung auf verschiedene Dienstleistungseinrichtungen ergibt sich das Problem, dass bei Dienstleistungseinrichtungen, welche einen ähnlichen Leistungskatalog umfassen, für ein Steuer- und Kontrollorgan schwer zu entscheiden ist, welche der zur Verfügung stehenden Dienstleistungseinrichtungen zur Erbringung einer Teilleistung der telemedizinischen Gesundheitsdienstleistung am besten geeignet ist. Des Weiteren gestaltet es sich häufig schwierig, den aktuellen Status einer zu erbringenden telemedizinischen Gesundheitsdienstleistung sowie die aktuell zuständige Stelle für die Erbringung einer anstehenden Teilleistung der telemedizinischen Gesundheitsdienstleistung zu ermitteln. Da also Verantwortlichkeiten und Zuständigkeiten häufig nicht eindeutig definiert sind und zugeordnet werden können, besteht die Gefahr, dass eine gleichbleibende hohe Qualität bei der Erbringung telemedizinischer Gesundheitsdienstleistungen nicht gewährleistet werden kann.

In der US 5,764,923 ist ein Verfahren beschrieben, wie Angestellte einer medizinischen Dienstleistungseinrichtung medizinischen Rat suchende Patienten durch den Einsatz von Informationstools in verschiedene Risikokategorien einordnen können ohne dabei eine medizinische Diagnose erstellen zu müssen, um den Patienten anschließend eine geeignete medizinische Behandlung ihres Leidens vermitteln zu können. Basierend auf der für einen Patienten zutreffenden Kategorie und den Bedürfnissen des Patienten wird der Zeitpunkt der medizinischen Behandlung, eine für die Behandlung des Patienten geeignete medizinische Einrichtung und die Art der Behandlung des Patienten empfohlen.

In der US 5,835,897 ist ein Verfahren beschrieben, welches Managern im Gesundheitswesen helfen soll, die Qualität und das Preis-/Leistungsverhältnis von Dienstleistungsanbietern im Gesundheitswesen bestimmen und vergleichen zu können. Dabei werden Patienten nach ihrer Krankheit und deren Behandlung klassifiziert. Basierend auf dieser Klassifizierung können die Leistungen verschiedener Dienstleistungsanbieter zur Behandlung der Krankheit verglichen und der zur Behandlung der Erkrankung bestmöglichste Anbieter ermittelt werden.

Aus der US 5,867,821 ist ein Kommunikationssystem bekannt, welches eine so genannte Masterbibliothek umfasst, auf der eine Vielzahl von Daten, u.a. Patientenakten, Bilddaten, Labordaten, pharmazeutische Daten etc., gespeichert sind. Auf diese Daten können verschiedene, mit der Masterbibliothek verbundene Rechner, die beispielsweise einer Krankenstation innerhalb eines Krankenhauses oder externen Ärzten zugeordnet sind, zugreifen.

In der US 5,706,441 ist ein Verfahren beschrieben, mit dem eine Art Rangliste von medizinischen Dienstleistungsanbietern auf einem bestimmten medizinischen Gebiet im Hinblick auf die Komplexität von von den medizinischen Dienstleitungsanbietern erbringbaren medizinischen Gesundheitsdienstleistung ermittelt werden kann. Hierzu werden von verschiedenen medizinischen Dienstleitungsanbietern ermittelte Datensätze zur Behandlung einer bestimmten Krankheit zusammengefasst und eine Bewertung der Einzelfälle nach dem Schwierigkeitsgrad der Behandlung vorgenommen. Anhand dieser Bewertung kann schließlich für einen Anbieter medizinischer Gesundheitsdienstleistungen, dessen medizinische Gesundheitsdienstleistungen bewertet worden sind, eine Aussage gemacht werden, welchen Komplexitätsgrad einer Erkrankung dieser medizinische Gesundheitsdienstleistungsanbieter in der Lage ist, adäquat zu behandeln.

Der Erfindung liegt die Aufgabe zugrunde, ein Verfahren der eingangs genannten Art derart anzugeben, dass in einfacher Weise eine Beauftragung für die Erbringung zumindest einer Teilleistung einer telemedizinischen Gesundheitsdienstleistung erfolgen kann und die Erbringung der Teilleistung überwacht werden kann.

Nach der Erfindung wird diese Aufgabe gelöst durch ein Verfahren zur Steuerung und Überwachung des Prozessablaufs einer zu erbringenden telemedizinischen Gesundheitsdienstleistung durch eine zentrale Datenverarbeitungseinrichtung, welche Gesundheitsdienstleistung eine oder mehrere verschiedene Teilleistungen umfassen kann, wobei eine bestimmte Teilleistung durch mehrere zur Verfügung stehende Dienstleistungseinrichtungen erbracht werden kann, welche Dienstleistungseinrichtungen jeweils über wenigstens einen Rechner verfügen, welche Rechner mit der zentralen Datenverarbeitungseinrichtung über ein Kommunikationsnetz kommunizieren können, aufweisend folgende Verfahrensschritte:
- basierend auf wenigstens einem Kriterium wenigstens eine automatisierte Beauftragung einer der zur Erbringung einer bestimmten Teilleistung zur Verfügung stehenden Dienstleistungseinrichtungen zur Erbringung der bestimmten Teilleistung der Gesundheitsdienstleistung durch die zentrale Datenverarbeitungseinrichtung, so dass bei einer mehrere Teilleistungen umfassenden telemedizinischen Gesundheitsdienstleistung eine Kette von Dienstleistungseinrichtungen automatisiert aufgestellt wird, von denen jede für die Erbringung einer bestimmten Teilleistung zuständig ist, und
- automatisierte Überwachung der Erbringung einer jeden Teilleistung durch die jeweils beauftragte Dienstleistungseinrichtung, in dem die zentrale Datenverarbeitungseinrichtung überwacht, ob die jeweilige Teilleistung entsprechend einer zeitlichen Vorgabe rechtzeitig von einem Rechner der jeweiligen Dienstleistungseinrichtung über das Kommunikationsnetz an die zentrale Datenverarbeitungseinrichtung übermittelt wird, und/oder ob die erbrachte jeweilige Teilleistung einer qualitativen Vorgabe genügt.

Nach der Erfindung liegt also in einer zentralen Datenverarbeitungseinrichtung, welche mit entsprechenden Datenverarbeitungseinrichtungen von Dienstleistungseinrichtungen einer Gesundheitsdienstleistungskette verbunden ist und die Steuerfunktion für den Prozessablauf übernimmt, wenigstens ein Kriterium, vorzugsweise ein Kriterienkatalog, vor, nach dem Zuständigkeiten für die Erbringung zumindest von Teilleistungen einer telemedizinischen Gesundheitsdienstleistung festgelegt werden können. Die zentrale Datenverarbeitungseinrichtung überprüft dabei unter Berücksichtigung von von einem Auftraggeber der telemedizinischen Gesundheitsdienstleistung vorgebrachten Wünschen und gestellten Bedingungen sowie unter Berücksichtigung von von den Dienstleistungseinrichtungen kommunizierten oder vorliegenden Leistungskatalogen, welche Dienstleistungseinrichtungen und in welcher Reihenfolge diese für die Erbringung der telemedizinischen Gesundheitsdienstleistung in Frage kommen. Sollte eine zu erbringende Gesundheitsdienstleistung nicht in verschiedene Teilleistungen untergliedert werden können oder eine Untergliederung nicht sinnvoll sein, legt die Datenverarbeitungseinrichtung anhand wenigstens eines Kriteriums eine zuständige Stelle für die Erbringung der Gesamtleistung fest. Auf diese Weise lassen sich eindeutig, abrufbar und nachvollziehbar Zuständigkeiten für die Erbringung einer telemedizinischen Gesundheitsdienstleitung festlegen, so dass eine eindeutige Verantwortlichkeit für die Erbringung einer Gesamtleistung existiert oder eindeutige Verantwortlichkeiten für die Erbringung von Teilleistungen existieren, wobei durch die gezielte Steuerung des Prozessablaufs einer Gesundheitsdienstleistung die Voraussetzungen geschaffen sind, eine gleichbleibend hohe Qualität bei der Erbringung telemedizinischer Gesundheitsdienstleistungen zu gewährleisten. Die zentrale Datenverarbeitungseinrichtung beauftragt dabei Dienstleistungseinrichtungen zur Erbringung von Teilleistungen und überwacht den Prozessablauf einer zu erbringenden telemedizinischen Gesundheitsdienstleistung auf die Einhaltung zeitlicher und/oder qualitativer Vorgaben für die zu erbringende Gesundheitsdienstleistung.

Eine Variante der Erfindung sieht vor, dass eine von der Datenverarbeitungseinrichtung beauftragte Dienstleistungseinrichtung die Übernahme der Bearbeitung der zentralen Datenverarbeitungseinrichtung bestätigen muss. Lehnt eine Dienstleistungseinrichtung die Erbringung einer Teilleistung der telemedizinischen Gesundheitsdienstleistung oder die Gesamtleistung ab, beauftragt die zentrale Datenverarbeitungseinrichtung eine geeignete alternative Dienstleistungseinrichtungen zur Erbringung der Gesamt- oder Teilleistungen. Auf diese Weise wird sichergestellt, dass die Gesundheitsdienstleistung tatsächlich erbracht wird bzw. dass keine Lücke in der Kette der zu erbringenden Teilleistungen entsteht. Dabei sind nicht nur Zuständigkeiten nachvollziehbar, sondern auch die Reihenfolge der Bearbeitung für die Erbringung von Teilleistungen einer telemedizinischen Gesundheitsdienstleistung.

Eine Variante der Erfindung sieht vor, dass die Beauftragung für die Erbringung zumindest einer Teilleistung einer telemedizinischen Gesundheitsdienstleistung nach einem fallspezifischen medizinischen Kriterium erfolgt. Ein derartiges Kriterium kann die zu bearbeitende Fallart, also beispielsweise die Aufnahme oder Auswertung von Mammographiebildern, Magnetresonanzbildsequenzen oder die Vornahme eines histologischen Schnittes sein. Ein anderes derartiges Kriterium kann ein Fallobjekt darstellen, also welches Organ oder Körperteil zu untersuchen ist. Des Weiteren gehören zu dieser Art von Kriterien die Komplexität des Falles, der Umfang der von einer Dienstleistungseinrichtung der Gesundheitsdienstleistungskette zu verarbeitenden Daten, beispielsweise der Umfang von A-namnesedaten oder die Anzahl von Bilddaten, sowie die Qualität der von einer Dienstleistungseinrichtung der Gesundheitsdienstleistungskette zu verarbeitenden Daten, insbesondere in Abhängigkeit davon, ob die gelieferten Daten manuell oder automatisch ausgewertet und entsprechende medizinische Entscheidungen davon abgeleitet werden sollen. Die fallspezifischen Kriterien sind also maßgeblich, um festzustellen, welche Dienstleistungseinrichtung überhaupt in der Lage ist, die geforderte medizinische Teilleistung zu erbringen.

Nach einer Variante der Erfindung ist ein Kriterium für die Beauftragung, die Person oder Institution, welche die telemedizinische Gesundheitsdienstleistung in Auftrag gibt. Berücksichtigt wird hierbei die Priorität, beispielsweise die vom Kunden vergebene Dringlichkeit sowie die Bedeutung bzw. Wichtigkeit des Kunden. Darüber hinaus stellt die Sensibilität ein Kriterium dar, d.h., ob bei der Erbringung der telemedizinischen Gesundheitsdienstleistung Daten mit höchster Geheimhaltungsstufe oder zu verschlüsselnde Daten anfallen. Ein weiteres mit der auftraggebenden Person oder Institution verknüpftes Kriterium liegt in der fachlichen Kompetenz des Auftraggebers, also ob eine renommierte Fachklinik oder ein unbekannter niedergelassener Arzt die telemedizinische Gesundheitsdienstleistungen in Auftrag gibt.

Nach einer Ausführungsform der Erfindung liegen Kriterien für die Beauftragung in der Art, in dem Umfang und in der Qualität des gewünschten Ergebnisses. Demnach wird als Kriterium berücksichtigt, ob ein Erstbefund erstellt werden soll, ob zu einem bereits vorliegenden Befund eine Zweitmeinung eingeholt werden soll oder ob ein detailliertes Gutachten gefordert wird.

Eine andere Ausführungsform der Erfindung sieht vor, dass die Beauftragung nach mitarbeiterspezifischen oder ausstattungsspezifischen Kriterien erfasst. Bei den mitarbeiterspezifischen Kriterien werden die Verfügbarkeit von Bearbeitern, deren Qualifikation und gegebenenfalls deren Zertifizierung, also ob es sich beispielsweise um renommierte Spezialisten oder bewährte Mitarbeiter handelt, sowie die Auslastung der Mitarbeiter, also wie viele Fälle ein Mitarbeiter bereits bearbeitet sowie der geschätzte Zeitpunkt der Ergebnislieferung berücksichtigt. Ebenfalls von Bedeutung für die Beauftragung sind vorhandene Arbeitsmittel und die technische Ausstattung einer Dienstleistungseinrichtung, damit adäquate Ergebnisse bei der Erbringung einer telemedizinischen Gesundheitsdienstleistung zu erwarten sind.

Nach einer Variante der Erfindung ist ein Kriterium für die Beauftragung einer Dienstleistungseinrichtung eine zeitliche Vorgabe. Berücksichtigt als Kriterium wird hierbei die geforderte oder versprochene Erledigungszeit für die zu erbringende telemedizinische Gesundheitsdienstleistung, und insbesondere, ob die Erledigungszeit eine Bearbeitung am Wochenende oder an Feiertagen oder sogar in Nachtschichten erforderlich macht. Insbesondere sind dabei bei über verschiedenen Ländern verteilten Dienstleistungseinrichtungen einer Gesundheitsdienstleistungskette Zeitverschiebung, Feiertage und unterschiedliche Wochentagsstrukturen zu beachten.

Gemäß einer Ausführungsform der Erfindung kann die Beauftragung auch nach einem technischen Kriterium erfolgen. Relevante Kriterien hierfür sind die Qualität und die Geschwindigkeit einer Telekommunikationsanbindung einer einer Dienstleistungseinrichtung zugeordneten Datenverarbeitungseinrichtung, also ob die Datenverarbeitungseinrichtung der Dienstleistungseinrichtung beispielsweise über Modem oder über eine Hochgeschwindigkeitsdatenverbindung beispielsweise mit der zentralen Datenverarbeitungseinrichtung der Gesundheitsdienstleistungskette verbunden ist. Weitere technische Kriterien stellen die Auslastung von Systemen einer Dienstleistungseinrichtung, deren Einsatz für die Erbringung einer Teilleistung einer Gesundheitsdienstleistung erforderlich ist, sowie Sicherheitsanforderungen dar. Bei der Festlegung der Zuständigkeit ist hinsichtlich von Sicherheitsanforderungen, insbesondere was den Datentransfer anbelangt, zu prüfen, inwieweit für die Bearbeitung einer Teilleistung einer telemedizinischen Gesundheitsdienstleistung betroffene Dienstleistungseinrichtungen in der Lage sind, erzeugte Daten beispielsweise zu verschlüsseln.

Gemäß einer weiteren Ausführungsform der Erfindung kann die Beauftragung auch nach wenigstens einem finanziellen Kriterium erfolgen. Zu berücksichtigen sind hierbei die Kosten, welche innerhalb einer Dienstleistungseinrichtung für die Erbringung einer Teilleistung einer telemedizinischen Gesundheitsdienstleistung anfallen, insbesondere für Expressbearbeitungen sowie eventuell vom Auftraggeber vorgegebene Kostenlimits.

Nach einer anderen Ausführungsform der Erfindung kann die Beauftragung auch nach rechtlichen Kriterien erfolgen, also inwieweit bestimmte telemedizinische Gesundheitsdienstleistungen in bestimmten Ländern überhaupt erbracht werden dürfen. Außerdem sind in den einzelnen Ländern vorhandene Datenschutzgesetze zu berücksichtigen.

Weitere Kriterien für die Beauftragung können die Sprache sein, in der das Ergebnis einer telemedizinischen Gesundheitsdienstleistung kommuniziert werden soll sowie politische und ethische Aspekte, wobei insbesondere Konflikte zwischen Völkern und Glaubensgemeinschaften, zu berücksichtigen sind. Des Weiteren kann ein Kriterium für Beauftragung darin liegen, inwieweit Ergebnisse einer telemedizinischen Gesundheitsdienstleistung weiter verwendet werden können, sei es für Studien oder sei es für Statistiken. So könnten beispielsweise Gesundheitsdienstleistungen, welche medizinische Leistungen erfordern, welche Gegenstand aktueller Studien sind, an Dienstleistungseinrichtung gegeben werden, welche die Studien durchführen.

Gemäß einer Variante der Erfindung ist es vorgesehen, bei Nichteinhaltung zeitlicher und/oder qualitativer Vorgaben einen Eskalationsprozess zu starten. Im Zuge des Eskalationsprozesses können Alarme an Dienstleistungseinrichtungen übermittelt werden, welche auf zeitliche Verzögerungen oder auf qualitative Mängel in Ergebnissen der geforderten Teilleistung hinweisen. Gemäß einer Variante der Erfindung ist es insbesondere vorgesehen, im Rahmen des Eskalationsprozesses die Beauftragung für die Erbringung zumindest einer Teilleistung einer Gesundheitsdienstleistung zu ändern, also von einer Dienstleistungseinrichtung, welche Vorgaben nicht eingehalten hat, auf eine andere Dienstleistungseinrichtung zu übertragen.

Nach einer Ausführungsform der Erfindung werden alle Beauftragungen und Ergebnisse, welche im Zuge der Erbringung einer telemedizinischen Gesundheitsdienstleistung erzielt werden, protokolliert. Dies ermöglicht es gemäß einer Variante der Erfindung den Prozessablauf bei der Erbringung einer telemedizinischen Gesundheitsdienstleistung unter Berücksichtigung der Beauftragungen und Ergebnisse zu visualisieren. Die Visualisierung kann beispielsweise derart ausgeführt sein, dass der gesamte Prozess, also alle Dienstleistungseinrichtungen, einer Gesundheitsdienstleistungskette, welche zusammen die telemedizinische Gesundheitsdienstleistung erbringen, dargestellt wird, wobei die bereits erzielten Teilergebnisse und Beauftragungen, der aktuelle Status und die noch zu durchlaufenden Dienstleistungseinrichtungen z.B. durch farblich Hervorhebung voneinander zu unterscheiden sind.

Insbesondere die Protokollierung ermöglicht es dabei gemäß einer Variante der Erfindung Prozessmessgrößen, beispielsweise die Bearbeitungszeit oder Qualitätsvorgaben, zu definieren, kontinuierlich oder stichprobenartig zu ermitteln und basierend auf den Prozessmessgrößen Schwachstellen des Prozessablaufes zu erkennen und diese durch Modifizierung des Prozessablaufes zu beseitigen. Darüber hinaus ist aufgrund der nachvollziehbaren Steuerungen des Prozesses also solches zertifizierbar.

Ein Ausführungsbeispiel der Erfindung ist in den beigefügten schematischen Zeichnungen dargestellt. Es zeigen:
- Fig. 1: vier mit einer zentralen Datenverarbeitungseinrichtung verbundene Dienstleistungseinrichtungen zur Erbringung von Teilleistungen einer telemedizinischen Gesundheitsdienstleistung, und
- Fig. 2: die Visualisierung eines Prozessablaufs der Erbringung einer telemedizinischen Gesundheitsdienstleistung.

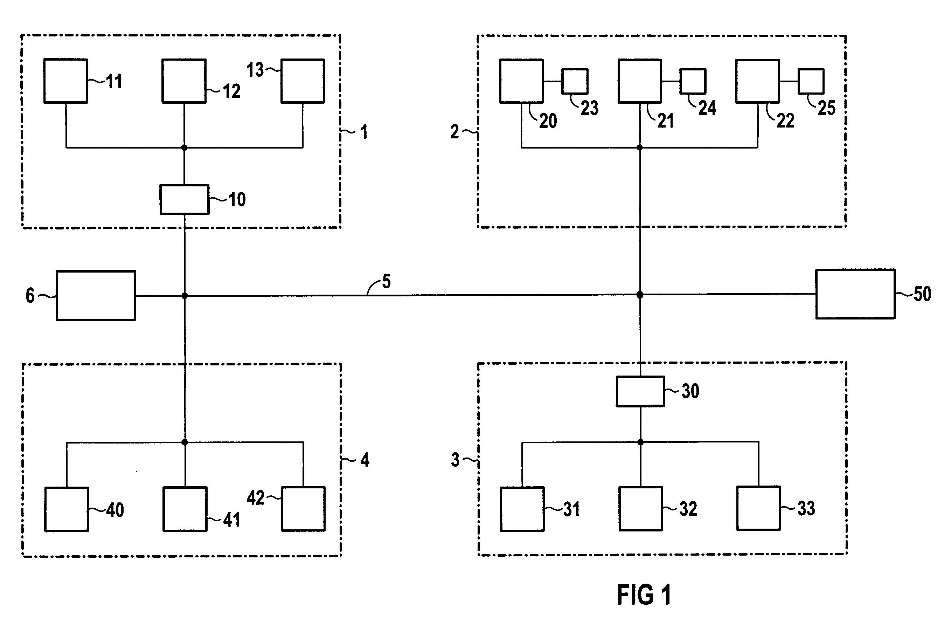

In der Fig. 1 sind exemplarisch vier Dienstleistungseinrichtungen 1 bis 4 zur Erbringung von Teilleistungen telemedizinischer Gesundheitsdienstleistungen sowie eine zentrale Datenverarbeitungseinrichtung 6 gezeigt, welche im Falle des vorliegenden Ausführungsbeispiels die Steuerung und Kontrolle von Prozessabläufen von zu erbringenden telemedizinischen Gesundheitsdienstleistungen übernimmt. Rechner der Dienstleistungseinrichtungen 1 bis 4 sind hierzu über ein Kommunikationsnetz 5 mit der zentralen Datenverarbeitungseinrichtung 6 verbunden. Im Falle des vorliegenden Ausführungsbeispiels wird die Steuerung und Kontrolle eines derartigen Prozessablaufes anhand einer telemedizinischen Gesundheitsdienstleistung, welche die Aufnahme und die Auswertung von ophthalmologischen Bildern umfasst, erläutert.

Die Dienstleistungseinrichtung 1 dient zur Aufnahme ophthalmologischer Bilder und umfasst einen Rechner 10, an den im Falle des vorliegenden Ausführungsbeispieles drei Geräte zur Aufnahme ophthalmologischer Bilder 11 bis 13 angeschlossen sind. Die Dienstleistungseinrichtung 2 dient ebenfalls zur Aufnahme ophthalmologischer Bilder und umfasst Rechner 20 bis 22. An jedem der Rechner 20 bis 22 ist jeweils ein Gerät 23 bis 25 zur Aufnahme ophthalmologischer Bilder angeschlossen. Die Rechner 20 bis 22 sind direkt über das Kommunikationsnetz 5 mit der zentralen Datenverarbeitungseinrichtung 6 verbunden und können mit dieser kommunizieren. Die Dienstleistungseinrichtung 3 umfasst einen direkt über das Kommunikationsnetz 5 mit der zentralen Datenverarbeitungseinrichtung 6 verbundenen Rechner 30, mit dem drei weitere Rechner 31 bis 33 verbunden sind. Die Dienstleistungseinrichtung 3 dient zur Auswertung von ophthalmologischen Bildern. Auch die Dienstleistungseinrichtung 4 dient zur Auswertung ophthalmologischer Bilder, wobei die Dienstleistungseinrichtung 4 drei Rechner 40 bis 42 umfasst, welche über das Kommunikationsnetz 5 direkt mit der zentralen Datenverarbeitungseinrichtung 6 verbunden sind.

Im Falle des vorliegenden Ausführungsbeispiels erhält die Datenverarbeitungseinrichtung 6 von einem Kunden, beispielsweise von einem Patienten oder von einer medizinischen Einrichtung, am 05.06.2001 um 10:00 Uhr den Auftrag zur Erbringung einer telemedizinischen Gesundheitsdienstleistung, welche im Falle des vorliegenden Ausführungsbeispiels Fundusaufnahmen an den Augen des Patienten und eine Auswertung der Aufnahmen umfasst. Der Auftrag wird von einem Rechner 50 des Kunden per E-Mail an die Datenverarbeitungseinrichtung 6 übermittelt. Der Kunde möchte das Ergebnis der Auswertung spätestens am 08.06.2001 um 15.00 Uhr und in deutscher Sprache geliefert haben. Der Kunde stellt keine besonderen Anforderungen an die Art, den Umfang und die Qualität des Ergebnisses, sondern ist vielmehr an ein möglichst kostengünstig Erstbefundung von Fundusaufnahmen interessiert. Seitens der Datenverarbeitungseinrichtung 6 besteht nun die Aufgabe, aus den für die Erbringung einer derartigen telemedizinischen Gesundheitsdienstleistung zur Verfügung stehenden Dienstleistungseinrichtungen 1 bis 4 diejenigen auszuwählen und für zuständig zu erklären, welche unter Berücksichtigung der Wünsche des Kunden am besten für die Durchführung der telemedizinischen Gesundheitsdienstleistung geeignet sind. Anhand eines Kriterienkatalogs legt die Datenverarbeitungseinrichtung 6 schließlich die Zuständigkeiten für die Erbringung von Teilleistungen der Gesundheitsdienstleistung fest. Zunächst überprüft die Datenverarbeitungseinrichtung 6 anhand von fallspezifischen Kriterien, welche der Dienstleistungseinrichtungen für die Aufnahme der Fundusaufnahme in Frage kommt. Für diese Aufgabe stehen der Datenverarbeitungseinrichtung 6 im Falle des vorliegenden Ausführungsbeispieles die Dienstleistungseinrichtungen 1 und 2 zur Verfügung.

Da es sich bei dem Kunden um einen gewöhnlichen Kunden handelt, müssen als Kriterien für die Festlegung der Zuständigkeit keine besonderen personenspezifischen oder institutionsspezifischen Kriterien, welche eine besonderes hochpriore oder sensible Bearbeitung erfordern könnten, berücksichtigt werden. Was die Art, den Umfang und die Qualität des gewünschten Ergebnisses anbelangt, so sind auch hierzu keine besonderen Kriterien zu berücksichtigen. Wie bereits erwähnt möchte der Kunde eine Erstbefundung der Fundusaufnahmen haben.

Was mitarbeiterspezifische und ausstattungsspezifische Kriterien anbelangt, so überprüft die Datenverarbeitungseinrichtung 6 welche der Dienstleistungseinrichtungen 1 und 2 in der Lage ist die Fundusaufnahmen durchzuführen. Im Falle des vorliegenden Ausführungsbeispiels ermittelt die Datenverarbeitungseinrichtung 6 über den Rechner 10 der Dienstleistungseinrichtung 1 die Auslastung der Geräte 11 bis 13 sowie die Verfügbarkeit der die Geräte 11 bis 13 bedienenden Personen für die nächsten drei Tage. Die Anfrage ergibt im Falle des vorliegenden Ausführungsbeispieles, dass die Fundusaufnahmen mit dem Gerät 12 am 06.06.2001 zwischen 10.00 und 12.00 Uhr durchgeführt werden können. Im Falle der Dienstleistungseinrichtung 2 kann die Datenverarbeitungseinrichtung 6 direkt durch eine an die Rechner 20 bis 22 der Geräte 23 bis 25 gerichtete Abfrage, die Verfügbarkeit der Geräte 23 bis 25 sowie die Verfügbarkeit der die Geräte 23 bis 25 bedienenden Personen ermitteln. Aus der Rückmeldung der Rechner 20 bis 22 registriert die Datenverarbeitungseinrichtung 6, dass die Fundusaufnahmen am 06.06.2001 zwischen 15:00 und 16:00 Uhr sowohl mit dem Gerät 21 als auch mit dem Gerät 22 durchgeführt werden können.

Was technische Kriterien, wie die Qualität und die Geschwindigkeit der Kommunikationsanbindung sowie Sicherheitsanforderungen, anbelangt, verfügen die Dienstleistungseinrichtungen 1 und 2 über dieselben Qualitätsstufe. Die Berücksichtigung finanzieller Kriterien, insbesondere des Wunsches des Kunden nach einer kostengünstigen Erbringung der Gesundheitsdienstleistung ergibt, dass die Fundusaufnahmen bei Beauftragung der Dienstleistungseinrichtung 1 günstiger als bei Beauftragung der Dienstleistungseinrichtung 2 sind. Weitere Kriterien, welche rechtlicher Aspekte, die verwendeten Sprache, politische und ethische Aspekte sowie Aspekte der Möglichkeit weiterer Fallauswertungen betreffen, sind hinsichtlich Fundusaufnahmen im Falle des vorliegenden Ausführungsbeispiels nicht zu berücksichtigen.

Aufgrund der finanziell günstigeren Erstellung der Fundusaufnahmen beauftragt die Datenverarbeitungseinrichtung 6 daher die Dienstleistungseinrichtung 1 die Fundusaufnahmen am 06.06.2001 um 10:00 Uhr für den Kunden zu erstellen und die Fundusaufnahmen bis 13:00 Uhr des 06.06.2001 an die Datenverarbeitungseinrichtung 6 zu übermitteln. Im Falle des vorliegenden Ausführungsbeispiels bestätigt der Rechner 10 der Dienstleistungseinrichtung 1 die Übernahme des Auftrages an die Datenverarbeitungseinrichtung 6, die den Kunden informiert und zur Aufnahme der Bilder für den 06.06.2001 10:00 Uhr in die Dienstleistungseinrichtung 1 einbestellt. Die Information des Kunden kann beispielsweise derart erfolgen, dass die Datenverarbeitungseinrichtung 6 ein E-Mail entsprechenden Inhaltes über das Kommunikationsnetz 5 an den Rechner 50 des Kunden sendet. Sollte der Kunde zu diesem Termin verhindert sein, was vorliegende nicht der Fall ist, ermittelt die Datenverarbeitungseinrichtung 6 einen Alternativtermin und teilt diesen dem Kunden mit.

In einem zweiten Schritt legt die Datenverarbeitungseinrichtung 6 aus den für die Bearbeitung der telemedizinischen Gesundheitsdienstleistung zur Verfügung stehenden Dienstleistungseinrichtungen 1 bis 4 die Zuständigkeit für die Auswertung der Fundusaufnahmen nach der Eignung für diese Aufgabe fest. Unter Berücksichtigung des fallspezifischen Kriteriums, Auswertung von Fundusaufnahmen von Augen, stehen hierfür die beiden Dienstleistungseinrichtungen 3 und 4 zur Verfügung. Da es sich bei dem Kunden, wie bereits erwähnt, um eine gewöhnliche Person handelt, bei der keine besonderen Prioritäten und Sensibilitäten zu berücksichtigen sind, spielen personenbezogene Kriterien auch für die Festlegung der Zuständigkeit für die Auswertung der Fundusaufnahmen keine Rolle. Da es sich bei der Auswertung um eine Erstauswertung von Fundusaufnahmen handelt, sind auch diesbezüglich keine besonderen Kriterien zu berücksichtigen.

Hinsichtlich der mitarbeiterspezifischen und ausstattungsspezifischen Kriterien ermittelt die Datenverarbeitungseinrichtung 6 durch Kontaktierung des Rechners 30, welcher der Personen zugeordneten Rechner 31 bis 33 für eine Auswertung von Fundusaufnahmen zwischen dem 06.06.2001 14:00 Uhr und dem 08.06.2001 12:00 Uhr zur Verfügung steht. Der Rechner 30 signalisiert der Datenverarbeitungseinrichtung 6, dass der Rechner 32 für eine derartige Auswertung am 07.06.2001 zwischen 09:00 und 11:00 Uhr zur Verfügung steht. Hinsichtlich der Dienstleistungseinrichtung 4 kann die Datenverarbeitungseinrichtung 6 direkt die Personen zugeordneten Rechner 40 bis 42 hinsichtlich ihrer Verfügbarkeit abfragen, wobei der Rechner 42 für eine Auswertung am 07.06.2001 zwischen 10:00 und 12:00 Uhr zur Verfügung steht.

Was technische Kriterien, finanzielle sowie rechtliche Aspekte anbelangt, so unterscheiden sich im Falle des vorliegenden Ausführungsbeispiels die Dienstleistungseinrichtungen 3 und 4 nicht. Im Falle des vorliegenden Ausführungsbeispiels spielen derartige Kriterien also keine Rolle. Zu berücksichtigen ist allerdings im Falle der Auswertung ein sprachliches Kriterium, da der Kunde die Auswertung und die aus der Auswertung abgeleiteten medizinischen Entscheidungen in deutscher Sprache erhalten möchte. Im Falle des vorliegenden Ausführungsbeispiels kann die Dienstleistungseinrichtung 3 die Auswertung und die davon abgeleiteten medizinischen Entscheidungen aufgrund der Kürze der Zeit allerdings nur in englischer Sprache liefern, weshalb die Dienstleistungseinrichtung 3 für die Auswertung der Fundusaufnahmen ausscheidet. Da keine weiteren politischen und ethischen Kriterien oder Kriterien, welche die Möglichkeiten der weiteren Fallauswertungen betreffen, gegen eine Beauftragung der Dienstleistungseinrichtung 4 sprechen, signalisiert die Datenverarbeitungseinrichtung 6 schließlich der Dienstleistungseinrichtung 4, speziell dem Rechner 42 der Dienstleistungseinrichtung 4, dass dieser bzw. die diesen Rechner bedienende Person für die Auswertung der Fundusaufnahmen zuständig ist. Die Auswertung der Fundusaufnahmen soll bis 07.06.2001 14:00 Uhr an die Datenverarbeitungseinrichtung 6 übermittelt werden. Im Falle des vorliegenden Ausführungsbeispiels signalisiert der Rechner 42 der Datenverarbeitungseinrichtung 6 seine Zuständigkeit, also die Übernahme des Auftrages. Demnach hat die Datenverarbeitungseinrichtung 6 anhand eines Kriterienkatalogs für die Gewinnung von Fundusaufnahmen und die Auswertung von Fundusaufnahmen eindeutig Zuständigkeiten abrufbar und nachvollziehbar festgelegt. Die Gesundheitsdienstleistungskette zur Erbringung dieser telemedizinischen Gesundheitsdienstleistung umfasst dabei die Dienstleistungseinrichtung 1 und die Dienstleistungseinrichtung 4.

Nach der Festlegung der Zuständigkeiten überwacht die Datenverarbeitungseinrichtung 6 automatisiert, ob die Fundusaufnahmen zu der vorbestimmten Zeit am 06.06.2001 durch die Dienstleistungseinrichtung 1 aufgenommen werden, indem die Datenverarbeitungseinrichtung 6 entsprechende Abfragen an den Rechner 10 der Dienstleistungseinrichtung richtet bzw. überwacht, ob die Fundusaufnahmen zu der geforderten Zeit an die Datenverarbeitungseinrichtung 6 gelangen. Im Falle des vorliegenden Ausführungsbeispiels werden die Fundusaufnahmen zu der vorgegebenen Zeit innerhalb der Dienstleistungseinrichtung 1 erzeugt und an die Datenverarbeitungseinrichtung 6 übermittelt. Nach Erhalt prüft die Datenverarbeitungseinrichtung 6 mittels eines Prüfprogramms automatisiert die Qualität der Fundusaufnahmen. Die Datenverarbeitungseinrichtung 6 ermittelt dabei die Bildqualität charakterisierende Parameter, wie beispielsweise die Bildhelligkeit und den Bildkontrast, und vergleicht diese mit Qualitätsvorgaben. Entspricht die Bildqualität wie im Falle des vorliegenden Ausführungsbeispiels den Vorgaben, übermittelt die Datenverarbeitungseinrichtung 6 die Fundusaufnahmen an den Rechner 42 der Dienstleistungseinrichtung 4 zur Auswertung der Fundusaufnahmen und überwacht, ob innerhalb der zeitlichen Vorgabe eine Auswertung der Fundusbilder erfolgt. Hätte die Datenverarbeitungseinrichtung 6 qualitative Mängel der Fundusaufnahmen festgestellt, hätten die Fundusaufnahmen nochmals durchgeführt werden müssen, was eine erneute Festlegung einer Zuständigkeit und eine Terminvereinbarung mit dem Kunden erfordert hätte.

Im Falle des vorliegenden Ausführungsbeispiels erfolgt durch einen Ausfall der den Rechner 42 bedienenden Person keine Auswertung der Fundusbilder bis zum 07.06.2001 14:00 Uhr. Die Datenverarbeitungseinrichtung 6 startet daher, nachdem keine Auswertung übermittelt wurde, zur Einhaltung des durch den Kunden vorgegebenen zeitlichen Rahmens einen Eskalationsprozess, bei dem im Falle des vorliegenden Ausführungsbeispiels überprüft wird, wer kurzfristig die Auswertung der Fundusaufnahmen vornehmen kann. Hinsichtlich kurzfristiger Auswertungen steht in der Dienstleistungseinrichtung 4 eine den Rechner 40 bedienende Person zur Verfügung, welche vorzugsweise hochpriore Fälle bearbeitet. Um hochpriore Fälle handelt es sich dann, wenn eine auswertende Stelle mit der Auswertung aufgrund einer unvorhergesehenen Komplexität des Falles überfordert ist oder wenn wie im vorliegenden Fall ein zeitkritischer Termin nicht eingehalten wurde. Im Falle des vorliegenden Ausführungsbeispiels existieren innerhalb der Dienstleistungseinrichtung 4 also unterschiedliche Kompetenzniveaus, was auch in den anderen Dienstleistungseinrichtungen der Fall sei kann.

Die Datenverarbeitungseinrichtung 6 beauftragt also den Rechner 40 bzw. die den Rechner 40 bedienende Person die noch nicht ausgewerteten Fundusaufnahmen möglichst umgehend auszuwerten und spätestens bis zum 07.06.2001 18:00 Uhr an die Datenverarbeitungseinrichtung 6 zu übermitteln. Im Falle des vorliegenden Ausführungsbeispiels übernimmt die den Rechner 40 bedienende Person den Auftrag zur Auswertung der Fundusaufnahmen, wodurch sich die Zuständigkeit geändert hat, was innerhalb der Datenverarbeitungseinrichtung 6 registriert wird. Die dem Rechner 40 zugeordnete Person wertet schließlich die Fundusaufnahmen aus, leitet medizinische Entscheidungen ab und übermittelt rechtzeitig einen entsprechenden Bericht an die Datenverarbeitungseinrichtung 6. Diese erstellt einen Gesamtbericht, welcher die Fundusaufnahmen sowie die Auswertung und die davon abgeleiteten medizinischen Entscheidungen enthält und übermittelt den Gesamtbericht bis zum 08.06.2001 15:00 Uhr an den auftraggebenden Kunden.

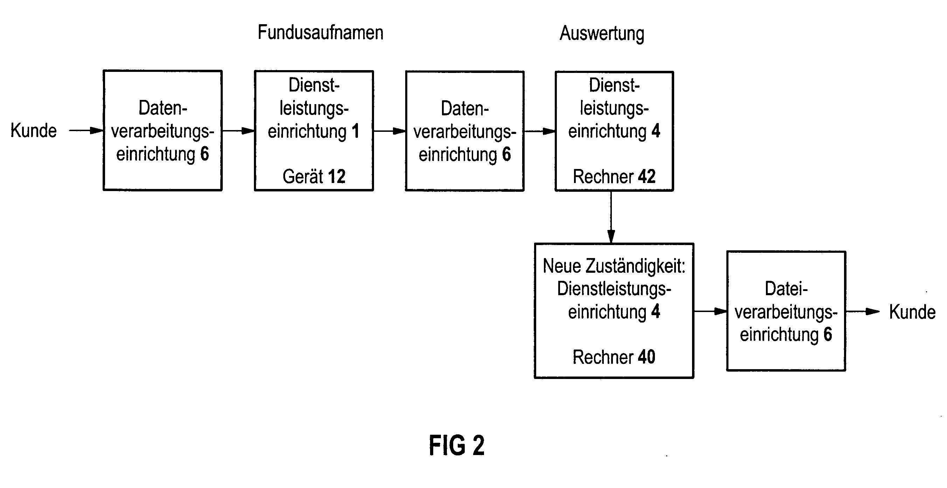

Im Zuge der Bearbeitung der telemedizinischen Gesundheitsdienstleistungen werden dabei von der Datenverarbeitungseinrichtung 6 alle Zuständigkeiten, der Wechsel von Zuständigkeiten sowie alle in den Dienstleistungseinrichtungen 1 und 4 erzielten Teilergebnisse protokolliert und gespeichert, so dass unter Verwendung einer Visualisierungssoftware der Prozessablauf visualisiert werden kann. Eine derartige Visualisierung ist für die erläuterte telemedizinische Gesundheitsdienstleistung in Fig. 2 dargestellt. Aus der Fig. 2 ist zu erkennen, dass die Fundusaufnahmen in der Dienstleistungseinrichtung 1 unter Verwendung des Gerätes 12 erzeugt wurden, welche über die Datenverarbeitungseinrichtung 6 an den Rechner 42 der Dienstleistungseinrichtung 4 weitergeleitet wurden. Aufgrund eines kurzfristigen Ausfalls der den Rechner 42 bedienenden Person hat die Zuständigkeit auf die den Rechner 40 bedienende Person gewechselt, welche die Auswertung der Fundusaufnahmen vorgenommen hat, und das Ergebnis der Auswertungen an die Datenverarbeitungseinrichtung 6 übermittelt hat. Die Datenverarbeitungseinrichtung 6 hat schließlich den Gesamtbericht erstellt und an den Kunden übermittelt.

Das erfindungsgemäße Verfahren wurde vorstehend am Beispiel der Aufnahme und Auswertung von Fundusaufnahmen beschrieben. Des Verfahren ist jedoch nicht auf die Steuerung einer derartigen Gesundheitsdienstleistung beschränkt. Vielmehr kann das Verfahren auch zur Steuerung von telemedizinischen Gesundheitsdienstleistungen eingesetzt werden, bei denen nur eine Gesamtleistung oder bei denen mehr als zwei Teilleistungen zu erbringen sind. Die Gesundheitsdienstleistungen müssen dabei nicht die Aufnahme und Auswertung von Bildern umfassen oder darauf beschränkt sein.

Die Festlegung von Zuständigkeiten muss im Übrigen nicht notwendigerweise von der Prüfung eines ganzen Kriterienkatalogs abhängig gemacht werden. Vielmehr kann die Prüfung auch nur eines wesentlichen Kriteriums für die Festlegung einer Zuständigkeit ausreichend sein.

Des weiteren muss die Bestätigung einer Zuständigkeit seitens einer Dienstleistungseinrichtung nicht notwendigerweise explizit erfolgen. Eine Bestätigung kann auch in der Ausführung einer Teilleistung oder Gesamtleistung liegen.

Im Unterschied zu dem vorstehend beschriebene Ausführungsbeispiel muss im Zuge eines Eskalationsprozesses nicht sofort die Zuständigkeit für die Erbringung einer Leistung geändert werden. Vielmehr kann einer mit einer Leistung in Verzug geratenen Dienstleistungseinrichtung der Verzug in Form eines Alarmsignals angezeigt und ein Ersatztermin für die Erbringung der Leistung vorgegeben oder vereinbart werden. Eine Änderung der Zuständigkeit würde erst dann vorgenommen, wenn auch der Ersatztermin nicht eingehalten würde. Zuständigkeiten können dabei innerhalb von Dienstleistungseinrichtungen oder zwischen Dienstleitungseinrichtungen geändert werden.

## Patentansprüche

1. Verfahren zur Steuerung und Überwachung des Prozessablaufs einer zu erbringenden telemedizinischen Gesundheitsdienstleistung durch eine zentrale Datenverarbeitungseinrichtung (6), welche Gesundheitsdienstleistung eine oder mehrere verschiedene Teilleistungen umfassen kann, wobei eine bestimmte Teilleistung durch mehrere zur Verfügung stehende Dienstleistungseinrichtungen (1 bis 4) erbracht werden kann, welche Dienstleistungseinrichtungen jeweils über wenigstens einen Rechner (10 bis 13, 20 bis 22, 30 bis 33, 40 bis 42) verfügen, welche Rechner (10 bis 13, 20 bis 22, 30 bis 33, 40 bis 42) mit der zentralen Datenverarbeitungseinrichtung (6) über ein Kommunikationsnetz (5) kommunizieren können, aufweisend folgende Verfahrensschritte:
- basierend auf wenigstens einem Kriterium wenigstens eine automatisierte Beauftragung einer der zur Erbringung einer bestimmten Teilleistung zur Verfügung stehenden Dienstleistungseinrichtungen (1 bis 4) zur Erbringung der bestimmten Teilleistung der Gesundheitsdienstleistung durch die zentrale Datenverarbeitungseinrichtung (6), so dass bei einer mehrere Teilleistungen umfassenden telemedizinischen Gesundheitsdienstleistung eine Kette von Dienstleistungseinrichtungen automatisiert aufgestellt wird, von denen jede für die Erbringung einer bestimmten Teilleistung zuständig ist, und
- automatisierte Überwachung der Erbringung einer jeden Teilleistung durch die jeweils beauftragte Dienstleistungseinrichtung (1 bis 4), in dem die zentrale Datenverarbeitungseinrichtung (6) überwacht, ob die jeweilige Teilleistung entsprechend einer zeitlichen Vorgabe rechtzeitig von einem Rechner (10 bis 13, 20 bis 22, 30 bis 33, 40 bis 42) der jeweiligen Dienstleistungseinrichtung (1 bis 4) über das Kommunikationsnetz (5) an die zentrale Datenverarbeitungseinrichtung (6) übermittelt wird, und/oder ob die erbrachte jeweilige Teilleistung einer qualitativen Vorgabe genügt.

2. Verfahren nach Anspruch 1, bei dem die beauftragte Dienstleistungseinrichtung (1 bis 4) die Übernahme der Bearbeitung bestätigen muss.

3. Verfahren nach Anspruch 1 oder 2, bei dem die Beauftragung nach einem fallspezifischen medizinischen Kriterium erfolgt.

4. Verfahren nach einem der Ansprüche 1 bis 3, bei dem ein Kriterium für die Beauftragung die Person oder Institution ist, welche die telemedizinische Gesundheitsdienstleistung initiiert.

5. Verfahren nach einem der Ansprüche 1 bis 4, bei dem Kriterien für die Beauftragung in der Art, in dem Umfang und in der Qualität des gewünschten Ergebnisses liegen.

6. Verfahren nach einem der Ansprüche 1 bis 5, bei dem die Beauftragung nach mitarbeiterspezifischen oder ausstattungsspezifischen Kriterien erfolgt.

7. Verfahren nach einem der Ansprüche 1 bis 6, bei dem ein Kriterium für die Beauftragung eine zeitliche Vorgabe ist.

8. Verfahren nach einem der Ansprüche 1 bis 7, bei dem die Beauftragung nach einem technischen Kriterium erfolgt.

9. Verfahren nach einem der Ansprüche 1 bis 8, bei dem die Beauftragung nach einem finanziellen Kriterium erfolgt.

10. Verfahren nach einem der Ansprüche 1 bis 9, bei dem die Beauftragung nach einem rechtlichen Kriterium erfolgt.

11. Verfahren nach einem der Ansprüche 1 bis 10, bei dem ein Kriterium für die Beauftragung die Sprache ist, in der das Ergebnis der telemedizinischen Gesundheitsdienstleistung kommuniziert werden soll.

12. Verfahren nach einem der Ansprüche 1 bis 11, bei dem die Beauftragung nach einem politischen oder ethischen Kriterium erfolgt.

13. Verfahren nach einem der Ansprüche 1 bis 12, bei dem ein Kriterium für die Beauftragung in einer Möglichkeit der weiteren Verwendung eines Ergebnisses der telemedizinischen Gesundheitsdienstleistung liegt.

14. Verfahren nach einem der Ansprüche 1 bis 13, bei dem bei Nichteinhaltung der zeitlichen und/oder der qualitativen Vorgaben ein Eskalationsprozess gestartet wird.

15. Verfahren nach Anspruch 14, bei dem im Rahmen des Eskalationsprozesses die Beauftragung für die Erbringung zumindest einer Teilleistung der Gesundheitsdienstleitung geändert wird.

16. Verfahren nach einem der Ansprüche 1 bis 15, bei dem alle Beauftragungen und Ergebnisse, welche im Zuge der Erbringung der telemedizinischen Gesundheitsdienstleistung erzielt werden, protokolliert werden.

17. Verfahren nach einem der Ansprüche 1 bis 16, bei dem der Prozessablauf unter Berücksichtigung der Beauftragungen und erzielter Ergebnisse visualisierbar ist.

18. Verfahren nach Anspruch 16 oder 17, bei dem basierend auf der Protokollierung Prozessmessgrößen ermittelt werden.
